Europäisches Patentamt

European Patent Office

Office européen des brevets

(11) Publication number:

**0 267 886**
**A1**

(12) # EUROPEAN PATENT APPLICATION

(21) Application number: **87850304.4**

(22) Date of filing: **12.10.87**

(51) Int. Cl.⁴: **G 01 N 33/564**

(30) Priority: **22.10.86 SE 8604511**

(43) Date of publication of application:
**18.05.88 Bulletin 88/20**

(84) Designated Contracting States:
**AT BE CH DE ES FR GB IT LI NL SE**

(71) Applicant: **Larsson, Anders**
**Tiundagatan 54 B**
**S-752 30 Uppsala (SE)**

(72) Inventor: **Larsson, Anders**
**Tiundagatan 54 B**
**S-752 30 Uppsala (SE)**

(74) Representative: **Nordén, Ake et al**
**AWAPATENT AB Box 7402**
**S-103 91 Stockholm (SE)**

(54) **Method for binding immune complexes.**

(57) The invention relates to a method for capturing or detecting immune complexes by means of an immunoadsorbent and/or immunodetector. The immune complexes are immune complexes preformed via complement factors or immune complexes formed in vivo. Bird antibodies, preferably chicken antibodies, are used as immunoadsorbent or immunodetector. The method comprises both capturing the immune complex by means of chicken antibodies and detecting the immune complex by means of chicken antibodies.

The invention further relates to the use of bird antibodies for capturing or detecting immune complexes in liquids containing such complexes.

The invention also relates to a composition for use in capturing or detecting immune complexes in individuals. The composition comprises an immunoabsorbent or immunodetector associated with suitable carriers in solid phase, such as a polymer material, or in liquid phase. The composition comprises bird antibodies as immunoadsorbent or immunodetector.

Fig. 2

## Description

## METHOD FOR BINDING IMMUNE COMPLEXES

### Field of the invention

The present invention relates to a new method for binding immune complexes in liquids to an immunoadsorbent and/or an immunodetector in order to demonstrate, quantify or isolate/remove said immune complexes. "Binding" of immune complexes here means capturing or detecting such complexes.

### Background of the invention

Since immune complexes have been found to play an important part in connection with diseases and for regulating the immune response of the body, considerable attention has lately been paid to the development of methods for determining immune complexes in patients suffering from various diseases.

Immune complexes are formed in body fluids or tissues by specific reactions between antigen molecules and antibody molecules. In these reactions, so-called complement factors (C1-C9) may participate. Some of these complement factors may, when activated under certain conditions, bind to immune complexes and be of importance for demonstrating, quantifying and isolating/removing the immune complexes.

A patient may exhibit immune response to a large number of antigens. Depending on the accessibility of the antigen, the antibodies produced may, together with the antigen, form immune complexes which primarily act so as to eliminate potential pathogens from the patient.

When the immune complex activates and binds complement factors, the immune complexes formed may be damaging to the patient and an immune complex disease arise. The factors decisive of such a development have not been clearly established, but it is obvious that essential factors are inter alia the type of antigen and antibody (IgG, IgM etc.), the molar ratio between them as well as the rate of production thereof. Further, in certain diseases, such as tumours, the presence of certain immune complexes may prevent the body from producing an immune response to the tumour.

Different methods are known for measuring immune complexes, e.g. methods based on complement binding, protein A binding, physical properties of the immune complex, conglutinin binding or binding to different receptors.

Several methods have been published for demonstrating, quantifying and isolating/removing immune complexes, see e.g. a survey by R. Blackstock, In Vitro Methods for Detection of Circulating Immune Complexes, Am. Clin. Lab. Sci. Vol. 11, No. 3, pp. 262-68, 1981. In this publication, reference is also made to a compilation made by the WHO of eighteen different detection methods, it being however pointed out that most of the methods available are both too unspecific for a successful clinical use and too complicated for use in small hospital units.

### The technical problem and its solution

Several of the known methods for assaying immune complexes are based on their ability to bind to receptors on cells from mammals. These cells, most often so-called Raji cells, must be kept in tissue culture, requiring special laboratories for carrying out the tests.

Antibodies from mammals are also used as immunoadsorbent. Such antibodies however suffer from certain drawbacks, namely that they can themselves activate the complement factors from mammals and also may disturb the system by being relatively similar to human antibodies. The same problem arises if the detector system for immune complexes consists of mammalian antibodies. Thus, in the use of mammalian antibodies as immunoadsorbent or immunodetector, these must be modified and split into Fab fragments, which complicates the process of capturing and detecting the immune complexes.

It has now been found that chicken antibodies have special properties and differ considerably from mammalian antibodies from the immunological point of view because of an entirely different reactivity pattern. This has been tested e.g. by means of the rheumatoid factor (RF) in tests with latex particles coated with antibodies from different mammals and from chickens. In these tests, the RF-positive serum reacted with all mammalian antibody-coated particles whereas not with the particles coated with chicken antibodies. This is of importance since patients with immune complex diseases often have a high RF frequency, which might thus give false results when using mammalian antibodies.

Nor do chicken antibodies exhibit any reactivity with protein A or protein G which are used as enzyme-labelled detector material in immune complex adsorbtion on antibody-coated ELISA plates (enzyme-linked immunosorbent assay). This lack of reactivity with protein A, protein G and the RF factor thus entails that no interference with these materials is had, and false test results are thus avoided as compared with the use of mammalian antibodies. Thus, chicken antibodies are both qualitatively and quantatively superior as adsorbents and detectors for immune complexes as compared with mammalian antibodies. Further, complement activation is avoided when using chicken antibodies since these do not activate human-complement factors. This is also an important advantage for the analysis result which is based on the demonstration of the result of a complement activation in vivo, for which reason further activation generated during analyses should thus be avoided.

In this context, the term "immunoadsorbent" relates to immune complex-binding antibodies on solid phase, for instance suitable adsorbent particles or surfaces. The term "immunodetector" here relates to antibodies in liquid phase for demonstrating immune complexes. The term "immune complex" here relates to the product which is formed of an antibody and an antigen, whether the complex has been produced in vivo or in vitro.

Further, as chicken antibodies can be produced from eggs, it is possible in a simple way to produce large amounts of chicken antibodies at a cost which is usually lower than the cost for corresponding antibodies produced in mammals. Moreover, whole mammalian antibodies cannot be used but must first be modified or split, as stated above.

Antibodies from birds, preferably chicken antibodies, thus provide a simple, rapid and reliable capturing and detecting method for use as immunoadsorbent and/or immunodetector. These antibodies have a different composition as compared with mammalian antibodies and, thus, need not be modified before use, since they do not disturb the system or bind complement factors from mammals.

As to the preparation and purification of antibodies from hens, reference is made to US-A 4,357,272 and 4,550,019. It is stated that such antibodies are used in immunological preparations for diagnostic purposes, but no use of immune complexes is indicated.

Description of the invention

The present invention thus relates to a method for capturing or detecting immune complexes by means of an immunoadsorbent and/or immunodetector in order to demonstrate, quantify or isolate/remove said immune complexes, which method is characterized in that the immune complexes are immune complexes preformed via complement factors or immune complexes formed in vivo, and that bird antibodies are used as immunoadsorbent or immunodetector. It is possible to use antibodies produced in birds and isolated from blood or egg, preferably chicken blood or chicken eggs.

The invention also relates to a composition comprising an immunoadsorbent and/or immunodetector together with a suitable carrier in solid phase or in liquid phase in order to demonstrate, quantify or isolate/remove said immune complexes, e.g. in individuals having a disease because of circulating immune complexes, said immunoadsorbent or immunodetector consisting of bird antibodies.

Prior methods for determining immune complexes generally comprise radioactively labelled antibody or antigen. Today, preference is given to detection by the so-called ELISA method, (enyme-linked immunosorbent assay) where the reactants are labelled with certain enymes instead of radioctive isotopes. Antibodies, in the present case generally chicken antibodies, may exist in solid phase, bound to agarose particles or to plastic (such as Microtitre® plates, ELISA plates). On addition of test solutions, these antibodies may then catch immune complexes existing therein, which thereafter bind to added enyme-labelled antibodies or enyme-labelled protein A from the bacterium Staphylococcus aureus, whereby they can be detected. Earlier known methods rely on binding to mammalian antibodies, causing however the problems stated above.

A very promising application of the invention is the use of chicken antibodies in so-called extracorporeal immunoadsorption therapy for treating patients with tumours or immune complex diseases. In such case, plasma from the patient is withdrawn for a certain period of time and is allowed to pass through a device for capturing the immune complexes, whereupon the immune complex-poor plasma is returned to the patient. In this manner, immune complex diseases can be alleviated and tumours caused to regress in that damaging immune complexes inhibiting the ability of the body to itself attack the tumour have been removed. By using chicken antibodies in this treatment, it is possible to achieve improved selectivity regarding the capture of immune complexes. In addition to this, the patient's complement system is not activated, and no injurious biologically active components are released or returned to the patient.

Realization of the invention

The method according to the invention is carried out as recited in the claims. In a preferred, non-limitative example, the method can be carried out in the following way (see Figs. 1a-1d):

1) Antibodies 1 from chickens (aC3, aC1$_q$ or aC4) are applied to Microtitre® plates 2 and adsorbed (see Fig. 1a).

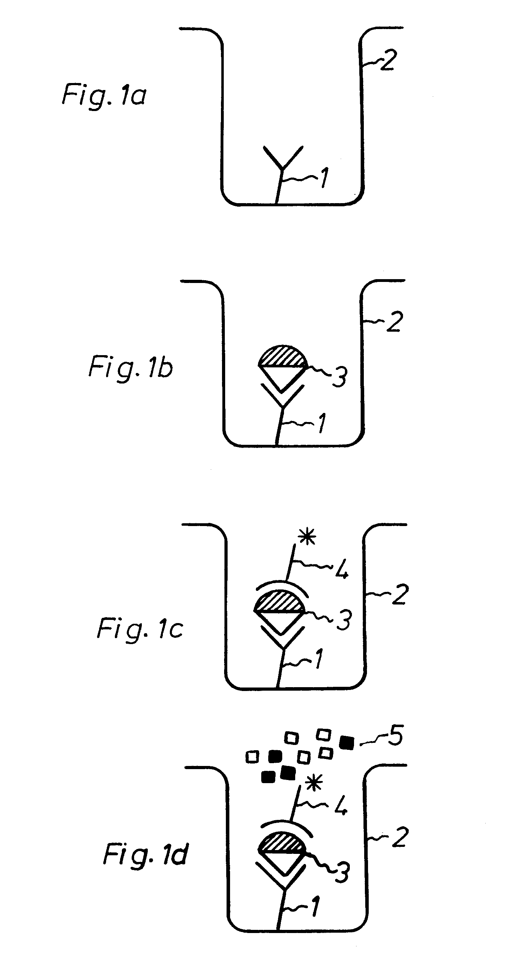

2) Samples containing immune complexes 3 are added (see Fig. 1b).

3) An enyme-labelled specific antibody material 4 (detector) is added (See Fig. 1c).

4) Detection of bound enyme-labelled material by colour analysis is carried out, giving a measurement of the amount of immune complex present (see Fig. 1d). A developing solution 5 is added, blank squares in Fig. 1d indicating parts not converted by the enzyme and filled squares indicating parts coloured by the enzyme. Reading can thereafter be effected with a spectrophotometer.

According to another preferred, non-limitative example, the method can be carried out in the following way:

1) Antibodies from chickens (aC3 or aC1$_q$) are coupled to particles of cross-linked agarose.

2) The agarose particles are packed in a column.

3) Samples containing immune complexes are applied to the column which is thereafter washed.

4) Immune complex bound to the antibodies is released by elution of the column.

5) Eluted material is freeze-dried and separated by electrophoresis on SDS-polyacrylamide gel for detecting and characterizing immune complex present.

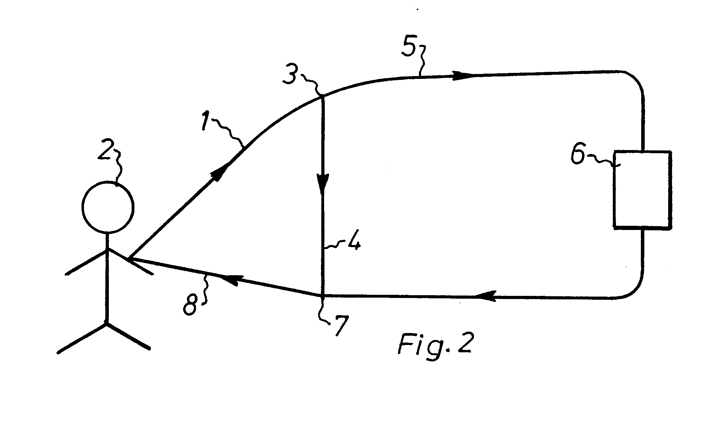

A variant of this example can be used for capturing immune complexes in extracorporeal circulation treatment (see Fig. 2). In that case, blood is withdrawn from the patient 2 through the conduit 1. At 3, the blood corpuscles are separated into the conduit 4, and the remaining plasma is conducted in the conduit 5 to a column 6 which is packed with particles of e.g. cross-linked agarose, to which chicken antibodies are coupled. The immune complex-poor plasma thereafter joins the separated blood corpuscles at 7 and is recycled to the patient through the conduit 8.

Example 1

1) Affinity-purified chicken anti-human C3 antibody is coupled to Sehparose® CL-4B (Pharmacia, Uppsala, Sweden) by the BrCN method.

2) The Sepharose particles are packed in a column and washed with 0.1 M HAc (acetic acid) and brought to equilibrium in PBS (0.15 M NaCl; 0.02 M $NaH_2PO_4$; 0.02% $Na N_3$; pH = 7.2).

3) The sample is applied to the column which is thereafter washed with PBS until all unbound protein has been washed off.

4) The proteins bound to the antibodies are released by eluting the column with 0.1 M HAc.

5) The material eluted with HAc is freeze-dried and separated by electrophoresis on SDS-polyacrylamide gel which is silver-coloured for identifying the eluted proteins.

Serum has been analyzed from normal individuals, patients with primary biliary cirrhosis (PBC), systemic lupus erythematosus (SLE) and rheumatic patients (RA). Here, the pattern of these patients differs from that found in normal individuals. In normal individuals, only normal C3 fragments were found, while in patients having PBC a large amount of additional protein bonds were found, which had not yet been identified.

Example 2

1) Micro-ELISA plates (Dynatech M129A) are coated with 200 µl of 20 µg affinity-purified chicken anti-human $C1_q$/ml in 0.02 M $NaH_2PO_4$; pH = 7.5. The plate is incubated for two hours at 37FC and thereafter washed three times with NaCl-Tween (0.9% NaCl; 0.05% Tween 20; 0.02% $NaN_3$). Any remaining sites in the plate are thereafter blocked with 0.1 mg Ovalbumin/ml in 0.02 M $NaH_2PO_4$; pH = 7.5. The plate is thereafter incubated for two hours at 37°C and washed three times with NaCl-Tween.

2) 150 µl PBS is added to all wells. 50 µl of serum samples or standards (heat-aggregated IgG diluted in normal serum) is thereafter added to the wells. To wells containing only 150 µl PBS is further added 50 µl PBS so that 200 µl is obtained in all wells. The plate is incubated for two hours at 37°C and thereafter washed three times with NaCl-Tween.

3) 200 µl protein A-alkaline phosphatase conjugate, diluted 1:2000 in PBS, is added to all wells, the plate is incubated for two hours at 37°C and thereafter washed three times with NaCl-Tween.

4) 200 µl of a developing solution is added to all wells (1 mg S-104 (Sigma)/ml in 1M diethanolamine; 0.5 mM $MgCl_2$; pH = 9.8), and the plate is incubated for 30 min. in darkness at room temperature. The development is interrupted with 50 µl 5 M NaOH, and the plate is read in a Titertek Multiskan.

In step 1, chicken anti-human C3 or chicken anti- human C4 may replace the $aC1_q$ antibody.

In step 3, it is also possible to use chicken anti-human IgG alkaline phosphatase conjugate instead of protein A conjugate.

The Table below lists the results from a test series according to the above-mentioned method with Bell's paresis patients tested with aC3 ELISA. In the Table, column I indicates patient number, "A" meaning acute sample (taken when the patient fell ill) and "K" meaning convalescent sample (taken on a repeat treatment at the hospital a few weeks after the patient had fallen ill). Column 2 indicates the ELISA value obtained, converted to µg heat-aggregated IgG used as standard. The upper limit for normal values has been set at 2.0 µg/ml, it being worth noting that none of the healthy patients exceeded this limit value. Column 3 indicates whether the patient was above or below 2 µg/ml.

The test series stated above shows that it is possible with the method according to the invention to qualitatively and quantitatively indicate in a rapid and simple manner very small amounts of immune complexes in the blood serum of a patient, which is most valuable in the continued diagnosis and assessment of the disease and of the measures to be taken for alleviating and curing the disease.

## TABLE

## Patient Material

| Sample No. | Microgram AIGG | IC-Serum |
|---|---|---|
| 2A | 3.02 | + |
| 2K | 3.62 | + |
| 3A | 1.74 | − |
| 3K | 2.26 | + |
| 4K | 1.96 | − |
| 5K | 2.64 | + |
| 7A | 1.74 | − |
| 8A | 2.71 | + |
| 14A | >50 | + |
| 23A | 7.12 | + |
| 29A | 1.96 | − |
| 33A | 1.66 | − |
| 34K | 2.49 | + |
| 35K | 3.32 | + |
| 37A | 1.05 | − |
| 39K | 1.58 | − |
| 41K | 2.79 | + |
| 43A | 3.47 | + |
| 43K | 2.42 | + |
| 44A | 2.42 | + |
| 44K | 4.08 | + |
| 45A | 3.40 | + |
| 46A | 15.7 | + |
| 46K | 12.8 | + |
| 47A | 14.6 | + |
| 48A | 20.3 | + |
| 48K | 21.7 | + |
| 49A | 1.65 | − |
| 49K | 1.32 | − |
| 51A | 2.35 | + |
| 51K | 2.05 | + |
| 52A | 3.28 | + |
| 52K | 3.69 | + |
| 53A | 2.35 | + |
| 54A | 1.32 | − |
| 54K | 2.87 | + |
| 55A | 1.84 | − |
| 56A | 1.84 | − |
| 56K | 2.35 | + |
| 57A | 1.54 | − |
| 57K | 1.44 | − |
| 58A | 3.59 | + |
| 58K | 3.39 | + |

| Sample No. | Microgram AIGG | IC-Serum |
|------------|----------------|----------|
| 59A | 1.75 | − |
| 61A | 3.69 | + |
| 61K | 2.66 | + |
| 63A | 2.56 | + |
| 69A | 4.10 | + |
| 71A | 1.33 | − |
| 71K | 0.82 | − |
| 74A | 6.47 | + |
| 74K | 10.0 | + |
| 75K | 0.93 | − |
| 76A | 4.00 | + |
| 76K | 4.10 | + |
| 78A | 1.66 | − |
| 78K | 1.88 | − |
| 80A | 1.50 | − |
| 82A | 2.13 | + |
| 82K | 2,27 | + |
| 83K | 1.96 | − |
| 84A | 0.87 | − |
| 84K | 0.75 | − |
| 85K | 0.43 | − |
| 86A | 2.94 | + |
| 86K | 2.34 | + |
| 88A | 0.48 | − |
| 88K | 0.45 | − |
| 89A | 1.12 | − |
| 89K | 0.97 | − |
| 90A | 1.33 | − |
| 90K | 1.44 | − |
| 93K | 0.41 | − |
| 94A | 2.02 | + |
| 95A | 0.16 | − |
| 80K | 1.95 | − |

# 0 267 886

## Normal Material

| Sample No. | Microgram AIGG | IC-Serum |
|---|---|---|
| 1 | 0.98 | – |
| 2 | 1.02 | – |
| 3 | 1.06 | – |
| 4 | 1.18 | – |
| 5 | 1.48 | – |
| 6 | 0.72 | – |
| 7 | 1.62 | – |
| 8 | 1.12 | – |
| 9 | 0.56 | – |
| 10 | 0.92 | – |
| 11 | 0.66 | – |
| 12 | 0.62 | – |
| 13 | 0.80 | – |
| 14 | 0.70 | – |
| 15 | 1.6 | – |
| 16 | 0.64 | – |
| 17 | 1.96 | – |

**Claims**

1. A method for capturing or detecting immune complexes by means of an immunoadsorbent and/or immunodetector, **characterized** in that the immune complexes are immune complexes preformed via complement factors or immune complexes formed in vivo, and that bird antibodies are used as immunoadsorbent or immunodetector.

2. Method as claimed in claim 1, **characterized** in that the bird antibodies comprise antibodies produced in birds.

3. Method as claimed in claim 1, **characterized** in that the bird antibodies comprise egg antibodies from birds.

4. Method as claimed in claims 1 and 2, **characterized** in that the bird antibodies comprise antibodies produced in chickens.

5. Method as claimed in claims 1 and 3, **characterized** in that the bird antibodies comprise egg antibodies from chickens.

6. Method for capturing immune complexes by means of an immunoadsorbent as claimed in claims 1-5, **characterized** in that the immune complex is captured by means of bird antibodies on solid phase.

7. Method as claimed in claim 6, **characterized** in that the capture of the immune complex by means of bird antibodies on solid phase is carried out on polymers, such as plastics, acrylamides and polysaccharides.

8. Method for detecting immune complexes by means of an immunodetector as claimed in claims 1-5, **characterized** in that the detection is carried out by means of an immunodetector comprising bird antibodies in liquid phase.

9. Method as claimed in claims 1-8, **characterized** in that the immune complex is captured by means of bird antibodies directed to antigen included in the complex or antibody included in the complex or complement factors included in the complex.

10. Method as claimed in claims 1-9, **characterized** in that the immune complex is captured by means of bird antibodies directed to complement factors.

11. Method as claimed in claims 1-10, **characterized** in that the immune complex is captured by means of bird antibodies directed to complement factors C3, $C1_q$ or C4.

12. The use of bird antibodies in the method as claimed in claims 1-11 for capturing or detecting immune complexes in liquids containing such complexes.

13. A composition for use in the method as claimed in claims 1-11 for capturing or detecting immune complexes in individuals having circulating immune complexes, said composition comprising an immunoadsorbent or immunodetector combined with suitable carriers in solid phase, such as a polymer material, or in liquid phase, **characterized** in that the immunoadsorbent or the immunodetector comprises bird antibodies.

Fig. 1a

Fig. 1b

Fig. 1c

Fig. 1d

*Fig. 2*

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | WO-A-8 102 469 (SCRIPPS CLINIC & RESEARCH FOUNDATION)<br>* Page 3, Table I; page 5, line 24 - page 6, line 17; page 7, lines 3-15; page 9, Table II; page 32, lines 9-29; page 40, lines 2-36 * | 7-13 | G 01 N 33/564 |
| X | POULTRY SCIENCE USA, vol. 60, no. 7, 1981, pages 1719-1720, US; R.B. RIMLER: "Methods for immunoelectrophoretic detection and quantitation of antigens with chicken or turkey antisera"<br>* Abstract * | 1-13 | |
| Y | EP-A-0 008 432 (BEHRINGWERKE AKTIENGESELLSCHAFT)<br>* Page 2, line 18 - page 3, line 3; page 4, lines 27-37; page 5, lines 30-39; page 16, line 1 - page 17, line 29 * | 1-13 | |
| Y | WO-A-8 201 593 (D. PARRATT)<br>* Page 5, lines 6-21; page 8, lines 17-24; page 13, line 3 - page 15, line 3 * | 1-13 | TECHNICAL FIELDS SEARCHED (Int. Cl. 4)<br><br>G 01 N |
| Y | EP-A-0 155 141 (TEIJIN LIMITED)<br>* Page 2, lines 9-27; page 3, lines 14-26; page 4, lines 24-34; page 19, line 3 - page 20, line 14 *<br>---      -/- | 1-13 | |

The present search report has been drawn up for all claims

| Place of search | Date of completion of the search | Examiner |
|---|---|---|
| THE HAGUE | 08-02-1988 | VAN BOHEMEN C.G. |

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 81, 9th December 1974, page 273, abstract no. 148835u, Columbus, Ohio, US; J.L. SONI et al.: "Serum antigen-antibody immune complex nephritis in malarious chickens" & IRCS LIBR. COMPEND. 1974, 2(4), 1259 | 1-13 | |
| Y | CHEMICAL ABSTRACTS, vol. 87, 19th December 1977, page 526, abstract no. 198995r, Columbus, Ohio, US; E.I. MORGAN et al.: "The role of antigen-antibody complexes in mediating immunologic unresponsiveness in the chicken" & J. IMMUNOL. 1977, 119(4), 1293-8 | 1-13 | |
| Y | CHEMICAL ABSTRACTS, vol. 99, 18th September 1983, page 471, abstract no. 20697e, Columbus, Ohio, US; R.B. RIMLER et al.: "Replacement of sodium chloride with dextran or polyethylene glycol for immunoprecipitation of lipopolysaccharide with antibodies in chicken or turkey sera" & VET. IMMUNOL. IMMUNOPATHOL. 1983, 4(4), 417-24 | 1-13 | TECHNICAL FIELDS SEARCHED (Int. Cl.4) |

The present search report has been drawn up for all claims

| Place of search | Date of completion of the search | Examiner |
|---|---|---|
| THE HAGUE | 08-02-1988 | VAN BOHEMEN C.G. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................................
& : member of the same patent family, corresponding
    document

EPO FORM 1503 03.82 (P0401)